(19) Europäisches Patentamt
European Patent Office
Office européen des brevets

(11) Veröffentlichungsnummer: **0 272 501**
**B1**

(12) **EUROPÄISCHE PATENTSCHRIFT**

(45) Veröffentlichungstag der Patentschrift: **23.05.90**

(51) Int. Cl.⁵: **C07C 51/29**, C07C 63/70

(21) Anmeldenummer: **87117624.4**

(22) Anmeldetag: **28.11.87**

(54) **Verfahren zur Herstellung von 4-(2'Chlorethyl) benzoesäure.**

(30) Priorität: **02.12.86 DE 3641099**

(43) Veröffentlichungstag der Anmeldung:
**29.06.88 Patentblatt 88/26**

(45) Bekanntmachung des Hinweises auf die Patenterteilung:
**23.05.90 Patentblatt 90/21**

(84) Benannte Vertragsstaaten:
**BE CH DE FR GB IT LI NL**

(56) Entgegenhaltungen:
**EP-A- 0 176 026**
**US-A- 2 969 385**
**US-A- 4 112 236**

**HOUBEN-WEYL "Methoden der Organischen Chemie", 4. Auflage, Band 8, Nr. 3, 1952, Seiten 415-416; "Sauerstoffverbindungen III", Georg Thieme Verlag, Stuttgart;**

(73) Patentinhaber: **HOECHST AKTIENGESELLSCHAFT, Postfach 80 03 20, D-6230 Frankfurt am Main 80(DE)**

(72) Erfinder: **Vorwerk, Edgar, Dr., Geisenheimer Strasse 88, D-6000 Frankfurt am Main 71(DE)**

EP 0 272 501 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft ein Verfahren zur Herstellung von 4-(2'-Chlorethyl)benzoesäure aus 4-(2'-Chlorethyl)acetophenon.

Ein solches Verfahren ist bereits bekannt. Nach der US-PS 2 969 385 wird 4-(2'-Chlorethyl)acetophenon mit einer alkalischen Natriumhypobromit-Lösung bei 0°C umgesetzt. Während der gesamten 4- bis 8-stündigen Reaktionsdauer wird diese Temperatur beibehalten. Die Umsetzung wird in einem Gemisch aus etwa 60 Teilen Wasser und 40 Teilen 1,4-Dioxan durchgeführt:

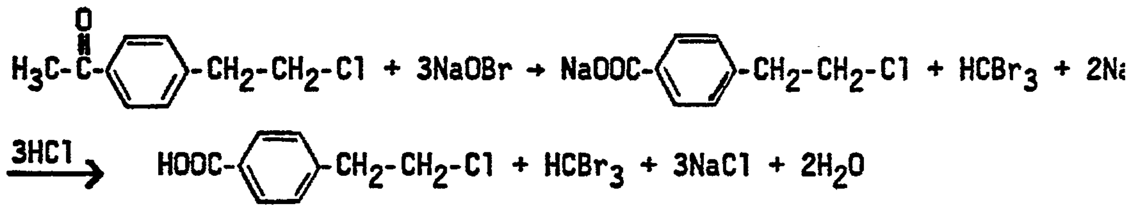

Das beschriebene Verfahren liefert die 4-(2'-Chlorethyl)-benzoesäure unter Einsatz einer sehr großen Brommenge : 3 Mol Brom = 479,5 g werden zur Herstellung von 3 Mol NaOBr benötigt, mit denen 1 Mol 4-(2'-Chlorethyl)acetophenon = 182,65 g zur Umsetzung gebracht werden. Bei diesem Verfahren bildet sich mehr von dem Abfallprodukt Bromoform als von dem Reaktionsprodukt 4-(2'-Chlorethyl)benzoesäure. Wegen der Verwendung eines Reaktionsgemisches aus 60 Teilen Wasser und 40 Teilen 1,4-Dioxan muß eine große Menge eines heute als bedenklich eingestuften Lösungsmittels eingesetzt werden, das aus diesem Gemisch nur schwer rückgewinnbar ist. Schließlich bedingt die Einhaltung der Reaktionstemperatur von 0°C einen erheblichen Kühlaufwand.

Das literaturbekannte Verfahren zur Herstellung der 4-(2'-Chlorethyl)benzoesäure ist also für eine technische Durchführung kaum geeignet.

Es bestand daher die Aufgabe, ein Verfahren zur Herstellung der genannten Carbonsäure zu entwickeln das von diesen Nachteilen frei ist. Diese Aufgabe wird durch die vorliegende Erfindung gelöst.

Ein Gegenstand der Erfindung ist demgemäß ein Verfahren zur Herstellung von 4-(2'-Chlorethyl)benzoesäure aus 4-(2'-Chlorethyl)acetophenon, dadurch gekennzeichnet, daß man 4-(2'-Chlorethyl)acetophenon mit einer wäßrig-alkalischen Lösung von Natriumhypochlorit und/oder Kaliumhypochlorit bei Temperaturen zwischen 25°C und 110°C umsetzt und dann die Lösung mit Hilfe einer starken anorganischen Säure ansäuert. Durch das Ansäuern wird die 4-(2'-Chlorethyl)-benzoesäure aus ihrem zunächst entstandenen Na- oder K-Salz in Freiheit gesetzt. Die Reaktionsgleichung lautet:

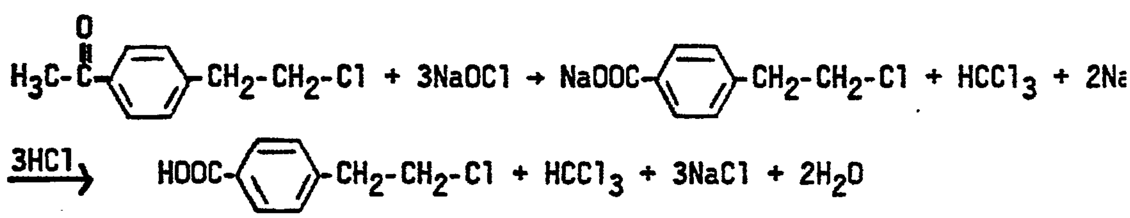

Die Umsetzung mit Hypochlorit kann in Gegenwart eines Lösungsvermittlers durchgeführt werden.

Als Lösungsvermittler eignen sich solche Substanzen, die sowohl mit dem 4-(2'-Chlorethyl)acetophenon als auch mit Wasser mischbar sind, gleichzeitig aber gegenüber Natrium- oder Kaliumhypochlorit chemisch inert sind. Diese Forderungen werden sowohl von den cycloaliphatischen Ethern, wie Tetrahydrofuran als auch von den aliphatischen Ethern, wie 1,2-Dimethoxyethan, Diethylenglykoldimethylether, Triethylenglykoldimethylether, sowie deren höheren Homologen erfüllt.

Die Umsetzung kann dann derart ausgeführt werden, daß man die wäßrig-alkalische Natrium- und/oder Kaliumhypochloritlösung mit der Lösung des 4-(2'-Chlorethyl)acetophenons in einem der zuvor benannten Lösungsvermittler (oder in einem beliebigen Gemisch derselben) versetzt und dieses Reaktionsgemisch ohne äußere Erwärmung rührt.

Nach einiger Zeit tritt eine merkliche Erwärmung ein, wobei sich die Temperaturzunahme mit steigender Temperatur beschleunigt. Vorzugsweise wird die Temperatur durch äußere Kühlung im Bereich von 28° bis 60°C gehalten.

Überraschenderweise verläuft die Umsetzung im wesentlichen ohne Bildung von 4-Vinylbenzoesäure, obwohl die Molekülstruktur der 4-(2'-Chlorethyl)benzoesäure eine leichte HCl-Abspaltung nahe-

legen würde.

Die Reaktion läßt sich aber auch ohne Zusatz eines Lösungsvermittlers durchführen. Dazu verrührt man die wäßrige alkalische Natrium- und/oder Kaliumhypochloritlösung allein mit dem 4-(2'-Chlorethyl)acetophenon; vorzugsweise begrenzt man durch Kühlung die Reaktionstemperatur wieder auf 28 bis 60°C. Bei sonst völlig analoger Reaktionsführung wie bei Gegenwart eines Lösungsvermittlers wird die Carbonsäure in praktisch gleicher Ausbeute und Reinheit erhalten. Bei dieser Art der Durchführung verlängert sich die Zeit zwischen Reaktionsanfang und dem Beginn der exothermen Reaktionsphase geringfügig.

Ist es aus verfahrenstechnischen Gründen erwünscht, ohne Kühlung zur arbeiten, so läßt sich dies durch entsprechend langsame Zugabe des Reaktanden (allein oder zusammen mit einem der erwähnten Lösungsvermittler) zu der vorgewärmten Natrium- und/oder Kaliumhypochloritlösung erreichen. Die exotherme Reaktion läßt sich bei dieser Verfahrensvariante ohne Kühlung abfangen, so daß die Reaktionstemperatur leicht in dem Bereich bis zu 60° C gehalten werden kann. Die jeweils für die exotherme Reaktion zur Verfügung stehende Substanzmenge ist durch die langsame Zugabe gering.

Die Menge an Lösungsvermittler liegt im allgemeinen bei 0 bis 50 Gew.-%, vorzugsweise bei 0 bis 10 Gew.-%.

Die Reaktionstemperatur liegt zwischen 25°C und der Siedetemperatur des Ansatzes (etwa 110°C), bevorzugt zwischen 28°C und 60°C.

Die Reaktionsdauer liege im allgemeinen zwischen 0,5 und 8 Stunden, vorzugsweise zwischen 1 und 5 Stunden.

Die Konzentrationen der Hypochloritlösungen liegen im allgemeinen zwischen 5 und 25 Gew.-% Natrium- bzw. Kaliumhypochlorit, bevorzugt zwischen 8 und 20 Gew.-%. Die Herstellung dieser Lösungen (etwa durch Einleiten von Chlor in wäßrige NaOH-Lösungen) wird in Gmelins Handbuch der Anorganischen Chemie: Chlor-Band, 1927, S. 265 ff. und der dort zitierten Literatur beschrieben.

Die 4-(2'-Chlorethyl)benzoesäure liegt nach der Umsetzung mit der Hypochloritlösung zunächst als Natrium- oder Kaliumsalz gelöst vor und wird durch Zugabe einer starken anorganischen Säure, wie Salzsäure, Bromwasserstoffsäure, Schwefelsäure oder Phosphorsäure bei pH-Werten von 1 bis 6, vorzugsweise bei pH 1 bis 3 ausgefällt.

Das Verfahren kann auch kontinuierlich durchgeführt werden.

Man kann natürlich, wenn man die Lösung des Na- oder K-Salzes statt der freien Säure erhalten will, auf die Zugabe der starken anorganischen Säure verzichten. Die Herstellung einer solchen Lösung ist ein weiterer Gegenstand der Erfindung.

Die 4-(2'-Chlorethyl)benzoesäure ist Ausgangspunkt für die Herstellung von 4-Vinylbenzoesäure durch HCl-Abspaltung, etwa mit Hilfe von Kaliumhydroxid in einem Lösungsmittel. 4-Vinylbenzoesäure ist ein interessantes Monomer zur Herstellung spezieller Polymere, wie z.B. beschrieben in Chemical Abstracts 65, 12362h (1966) (Copolymer mit speziellen Acrylamiden zur Eigenschaftsverbesserung von Gelatineschichten gegenüber Wasser); Chemical Abstracts 74, P 133057y (1971) (Copolymer mit Ethylacrylat und Laurylmethacrylat als Teil eines Entwicklers für die Elektrophotographie); US-PS 4 364 924 (Alkalisalze der Polyvinylbenzoesäure zur Verhinderung von Zahnbelag); Chemical Abstracts 98, P 207 565g (1983) (Copolymer mit Styrol als Teil eines photothermographischen Materials).

**Experimenteller Teil**

**Beispiel 1**

4,8 Mol Natriumhypochlorit (357,3 g) wurden in Form einer wäßrigen alkalischen Lösung (ca. 2260 ml Natriumhypochlorit-Lösung mit einem Gehalt von 0,158 g NaOCl pro ml und einem pH-Wert von 12 bis 13 durch NaOH) bei ca. 25°C vorgelegt. Dazu gab man 1,5 Mol 4-(2'-Chlorethyl)acetophenon (274 g), in 90 ml 1,2-Dimethoxyethan gelöst, und rührte das Reaktionsgemisch ohne zusätzliche Erwärmung. Das eingesetzte 4-(2'-Chlorethyl)acetophenon war (laut Gaschromatographie) ein 94 %iges Produkt; die restlichen 6 Prozent waren das isomere 2-(2'-Chlorethyl)acetophenon. Nach ca. einstündigen Rühren begann sich das Reaktionsgemisch merklich zu erwärmen. Durch äußere Kühlung hielt man die Reaktionstemperatur bei 32 bis 35°C. Damit dauerte die exotherme Reaktionsphase 1 bis 1,5 Stunden. Anschließend rührte man weitere 2 Stunden nach und ließ währenddessen die Temperatur im Reaktionsgefäß auf Raumtemperatur absinken. Die 4-(2'-Chlorethyl)benzoesäure - gelöst als Natriumsalz - wurde dann durch Salzsäurezugabe (ca. 475 ml konz. HCl) bei einem pH-Wert von 1 bis 2 ausgefällt. Man nutschte ab, trug die 4-(2'-Chlorethyl)benzoesäure in 1 l ca. 60°C warmes Wasser ein und verrührte den Feststoff für einige Minuten, um ihn von anhaftenden Salzsäure- und Salzresten zu befreien. Nach Abkühlung auf Raumtemperatur wurde erneut abgesaugt und anschließend das noch feuchte Reaktionsprodukt bei 50 bis 70°C unter vermindertem Druck für etwa 20 Stunden getrocknet.

270 g 4-(2'-Chlorethyl)benzoesäure (97,5 % d.Th.) mit einem Schmelzpunkt von 177 bis 180°C wurden erhalten. Gehalt der Probe: 95 % 4-(2'-Chlorethyl)benzoesäure; bestimmt durch GC nach Silylierung mit MSTFA (N-Methyl-N-trimethylsilyltrifluoracetamid)

Nach Umkristallisation aus Methanol betrug der Schmelzpunkt 187 bis 188°C, der Gehalt der Probe lag

bei über 99 % 4-(2'-Chlorethyl)benzoesäure.

1H-NMR-Daten (Lösungsmittel: DMSO-$D_6$)

| δ-Werte in ppm | Kopplungskonstanten | zugeordnete Protonen |
|---|---|---|
| Triplett 3,11 | 7 Hz | Ar-$CH_2$- |
| Triplett 3,90 | 7 Hz | -$CH_2$-Cl |
| Dublett 7,41 | 8 Hz | aromat. H, ortho zu Alkyl |
| Dublett 7,91 | 8 Hz | aromat. H, ortho zu Carboxy |
| Singulett 12,9 (breit) | | -COOH |

Beispiel 2

4,8 Mol Natriumhypochlorit (357,3 g) wurden in Form einer wäßrigen alkalischen Lösung (ca. 2335 ml Natriumhypochloritlösung mit einem Gehalt von 0,153 g NaOCl/ml und einem pH-Wert von 12 bis 13 durch NaOH) bei ca. 25°C vorgelegt. Dazu gab man 1,5 Mol 4-(2'-Chlorethyl)acetophenon (274 g mit einem Gehalt von 94 Gew.-%, wie in Beispiel 1), in 90 ml 1,2-Dimethoxyethan gelöst, und rührte das Reaktionsgemisch ohne zusätzliche Erwärmung.

Nach ca. einstündigem Rühren begann sich das Reaktionsgemisch merklich zu erwärmen. Durch äußere Kühlung hielt man die Reaktionstemperatur zwischen 38° und 42°C. Damit dauerte die exotherme Reaktionsphase ca. eine Stunde. Anschließend rührte man weitere 2 Stunden nach und ließ dabei die Temperatur auf Raumtemperatur absinken. Bei einem pH-Wert von 1 bis 2 wurde die 4-(2'-Chlorethyl)benzoesäure aus ihrem Natriumsalz durch Salzsäurezugabe freigesetzt. Man nutschte ab, trug die 4-(2'-Chlorethyl)benzoesäure in 1 l Wasser bei 60°C ein, verrührte einige Minuten und saugte nach Abkühlung auf Raumtemperatur erneut ab. Das feuchte Reaktionsprodukt wurde unter vermindertem Druck bei 50 bis 70°C für 20 Stunden getrocknet.

264 g 4-(2'-Chlorethyl)benzoesäure (95,3 % d.Th.) mit einem Schmelzpunkt von 179 bis 182°C wurden erhalten. Gehalt der Probe: 95 % 4-(2'-Chlorethyl)benzoesäure; bestimmt durch GC nach Silylierung mit MSTFA.

**Beispiel 3**

4,8 Mol Natriumhypochlorit (357,3 g) wurden in Form einer wäßrigen NaOH-alkalischen Lösung (ca. 2206 ml Natriumhypochloritlösung mit einem Gehalt von 0,162 g NaOCl/ml und einem pH-Wert von 12 bis 13) bei ca. 25°C vorgelegt. Dazu gab man 1,5 Mol 4-(2'-Chlorethyl)acetophenon (274 g mit einem Gehalt von 94 Gew.-%, wie in Beispiel 1), in 90 ml 1,2-Dimethoxyethan gelöst, und rührte das Reaktionsgemisch ohne zusätzliche Erwärmung. Nach ca. einstündigem Rühren begann sich das Reaktionsgemisch merklich zu erwärmen. Durch äußere Kühlung hielt man die Reaktionstemperatur zwischen 45 und 52°C.

Damit dauerte die exotherme Reaktionsphase etwa 0,5 bis 1 Stunde. Anschließend rührte man 2 Stunden nach und ließ währenddessen die Temperatur des Reaktionsgemisches auf Raumtemperatur absinken. Die Aufarbeitung dieses Ansatzes erfolgte wie in den Beispielen 1 und 2 beschrieben.

268 g 4-(2'-Chlorethyl)benzoesäure (96,8 % d.Th.) mit einem Schmelzpunkt von 178 bis 181°C wurden erhalten. Gehalt der Probe: 94 % 4-(2'-Chlorethyl)benzoesäure; bestimmt durch GC nach Silylierung mit MSTFA.

**Beispiel 4**

1,6 Mol Natriumhypochlorit (119,1 g) wurden in Form einer wäßrigen NaOH-alkalischen Lösung (ca. 713 ml Natriumhypochloritlösung mit einem Gehalt von 0,167 g NaOCl/ml und einem pH-Wert von 12 bis 13) bei ca. 25°C vorgelegt. Dazu gab man 0,5 Mol 4-(2'-Chlorethyl)acetophenon (91,3 g mit einem Gehalt von 94 Gew.-%, wie in Beispiel 1), in 30 ml 1,2-Dimethoxyethan gelöst, und rührte das Reaktionsgemisch ohne zusätzliche Erwärmung. Nach ca. einstündigem Rühren begann sich das Reaktionsgemisch merklich zu erwärmen. Ohne jegliche Kühlung ließ man die Temperatur ansteigen. Dies erfolgte zunächst sehr langsam, beschleunigte sich aber mit der Temperaturzunahme. Oberhalb von ca. 50°C stieg die Temperatur dann innerhalb von wenigen Minuten auf Siedetemperatur an, und das Reaktionsgemisch kochte auf. Die heftige exotherme Reaktion dauerte in diesem Fall lediglich einige Minuten. Danach ließ man die Innentemperatur auf Raumtemperatur absinken und rührte noch etwas nach, was insgesamt 2 Stunden dauerte. Durch Salzsäurezugabe fällte man bei einem pH-Wert von 1 bis 2 das Reaktionsprodukt aus und arbeitete es in der in Beispiel 1 beschriebenen Weise auf.

Nach Trocknung bei etwa 50 bis 60°C unter vermindertem Druck für 20 Stunden wurden 88,2 g weißer

Feststoff erhalten. Dieses Reaktionsprodukt zeigte keinen einheitlichen Schmelzpunkt; es erweichte bereits bei 170°C und schmolz zwischen ca. 174 und 181°C, ohne daß eine klare Schmelze erhalten wurde. Im 1H-NMR-Spektrum des Reaktionsproduktes erkannte man zusätzlich zu den üblichen Signalen der 4-(2'-Chlorethyl)benzoesäure (siehe Beispiel 1) deutlich die Signale der 4-Vinylbenzoesäure, gebildet durch HCl-Eliminierung.

1H-NMR-Daten (Lösungsmittel: DMSO-$D_6$) der 4-Vinylbenzoesäure:

| δ-Werte in ppm | Kopplungskonstanten | zugeordnete Protonen |
|---|---|---|
| Dublett-Dublett 5,41 | 10 Hz + 1 Hz | Ar-C=C-H, trans |
| Dublett-Dublett 5,97 | 18 Hz + 1 Hz | Ar-C=C-H, cis |
| Dublett-Dublett 6,82 | 18 Hz + 10 Hz | Ar-CH=C |
| Dublett (breit) 7,60 | 8 Hz | aromat. H, ortho zu Vinyl |
| Dublett (breit) 7,94 | 8 Hz | aromat. H, ortho zu Carboxy |
| Singulett (breit) 12,95 | | -COOH |

Gehalt der Probe gemäß GC nach Silylierung mit MSTFA:
88 % 4-(2'-Chlorethyl)benzoesäure
6 % 4-Vinylbenzoesäure
ca. 6 % weitere Substanzen.

**Beispiel 5**

4,6 Mol Natriumhypochlorit (342,4 g) wurden in Form einer wäßrigen NaOH-alkalischen Lösung (ca. 2167 ml Natriumhypochloritlösung mit einem Gehalt von 0,158 g NaOCl/ml und einem pH-Wert von 12 bis 13) bei ca. 25°C vorgelegt. Dazu gab man 1,5 Mol 4-(2'-Chlorethyl)acetophenon (274 g mit einem Gehalt von 94 Gew.-%, wie in Beispiel 1), in 90 ml Diethylenglykoldimethylether gelöst, und rührte das Reaktionsgemisch ohne zusätzliche Erwärmung. Nach ca. einstündigem Rühren begann sich das Reaktionsgemisch merklich zu erwärmen, und man hielt durch äußere Kühlung die Reaktionstemperatur zwischen 34 und 45°C. Die exotherme Reaktionsphase dauerte etwa eine Stunde. Anschließend wurde für weitere zwei Stunden nachgerührt, die Innentemperatur sank währenddessen auf Raumtemperatur ab. Die 4-(2'-Chlorethyl)benzoesäure wurde durch Salzsäurezugabe bei einem pH-Wert von 1 bis 2 ausgefällt, abgenutscht und mit Wasser nachgewaschen. Nach der Trocknung wurden 269 g 4-(2'-Chlorethyl)benzoesäure (97,1 % d.Th.) mit einem Schmelzpunkt von 177 bis 180°C erhalten.

Gehalt der Probe: 94 % 4-(2'-Chlorethyl)benzoesäure, bestimmt durch GC nach Silylierung mit MSTFA.

**Beispiel 6**

4,7 Mol Natriumhypochlorit (349,9 g) wurden in Form einer wäßrigen NaOH-alkalischen Lösung (ca. 2200 ml Natriumhypochloritlösung mit einem Gehalt von 0,159 g NaOCl/ml sowie einem pH-Wert von 12 bis 13) bei 25°C vorgelegt. Dazu gab man 1,5 Mol 4-(2'-Chlorethyl)acetophenon (274 g mit einem Gehalt von 94 Gew.-%, wie in Beispiel 1), in 90 ml Tetrahydrofuran gelöst, und rührte das Reaktionsgemisch ohne zusätzliche Erwärmung. Nach ca. einstündigem Rühren erwärmte sich das Reaktionsgemisch merklich, und man hielt durch äußere Kühlung die Reaktionstemperatur zwischen 35 und 40°C. Nach dem Ende der exothermen Reaktionsphase ließ man die Innentemperatur auf Raumtemperatur absinken und isolierte die 4-(2'-Chlorethyl)benzoesäure, wie in den Beispielen 1 und 2 beschrieben.

263 g 4-(2'-Chlorethyl)benzoesäure (95 % d.Th.) wurden nach Trocknung erhalten; der Schmelzpunkt betrug 178 bis 180°C. Gehalt der Probe: 94 % 4-(2'-Chlorethyl)benzoesäure; besimmt durch GC nach Silylierung mit MSTFA.

**Beispiel 7**

3,2 Mol Natriumhypochlorit (238,2 g) wurden in Form einer wäßrigen NaOH-alkalischen Lösung bei 25°C vorgelegt (ca. 1517 ml einer Natriumhypochloritlösung mit einem Gehalt von 0,157 g NaOCl/ml und einem pH-Wert von 12 bis 13). Dazu gab man 1 Mol 4-(2'-Chlorethyl)acetophenon (182,65 g mit einem Gehalt von 94 Gew.-%, wie im Beispiel 1). Das Gemisch wurde ohne zusätzliche Erwärmung gerührt. Nach ca. 1,5-stündigen Rühren begann die Temperatur des Ansatzes merklich anzusteigen, und man hielt durch äußere Kühlung die Reaktionstemperatur zwischen 37° und 40°C. Damit dauerte die exotherme Reaktionsphase etwa eine Stunde. Anschließend wurde für 2 Stunden nachgerührt, währenddessen die Innentemperatur auf Raumtemperatur absank. Die Isolierung, Reinigung und Trocknung des Produktes erfolgte wie in den Beispielen 1 und 2 beschrieben.

Nach der Trocknung verblieben 176 g 4-(2'-Chlorethyl)benzoesäure (95,3 % d.Th.) mit einem Schmelzpunkt von 178 bis 182°C.

Gehalt der Probe: 95 % 4-(2'-Chlorethyl)benzoesäure; bestimmt durch GC nach Silylierung mit MSTFA.

**Beispiel 8**

3,2 Mol Natriumhypochlorit (238,2 g) wurden in Form einer wäßrigen NaOH-alkalischen Lösung - vorgewärmt auf 40°C - vorgelegt (ca. 1567 ml einer Natriumhypochloritlösung mit einem Gehalt von 0,152 g NaOCl/ml und einem pH-Wert von 12 bis 13). Dazu tropfte man eine Lösung von 1 Mol 4-(2'-Chlorethyl)acetophenon (182,65 g mit einem Gehalt von 94 Gew.-%, wie im Beispiel 1), in 80 ml 1,2-Dimethoxyethan gelöst, im Verlauf von 2,5 Stunden unter ständigem Rühren gleichmäßig zu.

Etwa eine halbe Stunde nach Zugabebeginn beobachtete man einen Temperaturanstieg über 40°C hinaus. Durch gelinde äußere Kühlung gelang es leicht, die Reaktionstemperatur im Bereich von 40 bis 45°C zu halten, da durch die langsame Zugabegeschwindigkeit jeweils nur eine geringe Substanzmenge für die exotherme Reaktion zur Verfügung stand. Nach dem Zugabeende und dem Abklingen der exothermen Reaktionsphase rührte man 2 Stunden bei etwa 35°C nach. Die 4-(2'-Chlorethyl)benzoesäure wurde durch Salzsäurezugabe aus ihrem Natriumsalz bei pH-Werten von 1 bis 2 ausgefällt. Man saugte ab und wusch mit Wasser nach. Nach der Trocknung bei 50 bis 60°C unter vermindertem Druck für etwa 20 Stunden wurden 177 g 4-(2'-Chlorethyl)benzoesäure (95,8 % d.Th.) mit einem Schmelzpunkt von 177 bis 183°C erhalten.

Gehalt der Probe: 93 % 4-(2'-Chlorethyl)benzoesäure; bestimmt durch GC nach Silylierung mit MSTFA.

Nach Umkristallisation aus Methanol betrug der Schmelzpunkt 186 bis 187°C; die umkristallisierte 4-(2'-Chlorethyl)benzoesäure war ein mindestens 99 %iges Produkt.

**Beispiel 9**

(Umsetzung der 4-(2'-Chlorethyl)benzoesäure zur 4-Vinylbenzoesäure)

In eine auf 70°C vorgewärmte Lösung von 6,0 Mol Kaliumhydroxid (336,7 g) in 1000 ml Methanol wurden portionsweise, aber zügig, 2,0 Mol 4-(2'-Chlorethyl)benzoesäure (369,2 g) unter ständigem Rühren eingetragen, wobei eine exotherme Reaktion beobachtet wurde. Anschließend erhitzte man 5 Stunden zum Sieden. Nach beendeter Umsetzung wurde das Reaktionsgemisch auf etwa 0°C abgekühlt und das ausgefallene Kaliumsalz der 4-Vinylbenzoesäure abgenutscht. Dieses löste man in 1800 ml Wasser und setzte die 4-Vinylbenzoesäure durch Salzsäurezugabe bei einem pH-Wert von 1 bis 2 frei. Es wurde abgesaugt, mit Wasser nachgewaschen und die noch nasse 4-Vinylbenzoesäure in Methyl-tert.-butylether aufgenommen. Nach Entfernung des Restwassers trocknete man über Natriumsulfat, engte ein und erhielt 249,7 g 4-Vinylbenzoesäure (84,3 % d.Th.) mit einem Schmelzpunkt von 136 bis 139°C (unter Zersetzung.

Die 1H-NMR-Daten waren identisch mit den in Beispiel 4 angegebenen Daten für die dort als Nebenprodukt entstandene 4-Vinylbenzoesäure.

Gehalt der Probe: 96 % 4-Vinylbenzoesäure; bestimmt durch GC nach Silylierung mit MSTFA.

**Patentansprüche**

1. Verfahren zur Herstellung von 4-(2'-Chlorethyl)benzoesäure aus 4-(2'-Chlorethyl)acetophenon, dadurch gekennzeichnet, daß man 4-(2'-Chlorethyl)acetophenon mit einer wäßrig-alkalischen Lösung von Natriumhypochlorit und/oder Kaliumhypochlorit bei Temperaturen zwischen 25°C und 110°C umsetzt und dann die Lösung mit Hilfe einer starken anorganischen Säure ansäuert.

2. Verfahren nach Anspruch 1, dadurch gekennzeichnet, daß man die Umsetzung bei Temperaturen zwischen 28°C und 60°C durchführt.

3. Verfahren zur Herstellung einer wäßrigen Lösung des Natrium- und/oder Kaliumsalzes der 4-(2'-Chlorethyl)benzoesäure aus 4-(2'-Chlorethyl)acetophenon, dadurch gekennzeichnet, daß man 4-(2'-Chlorethyl)acetophenon mit einer wäßrig-alkalischen Lösung von Natriumhypochlorit und/oder Kaliumhypochlorit bei Temperaturen zwischen 25°C und 110°C umsetzt.

4. Verfahren nach Anspruch 3, dadurch gekennzeichnet, daß man die Umsetzung bei Temperaturen zwischen 28°C und 60°C durchführt.

**Claims**

1. A process for the preparation of 4-(2'-chloroethyl)benzoic acid from 4-(2'-chloroethyl)acetophenone, which process comprises reacting 4-(2'-chloroethyl)acetophenone with an aqueous alkaline solution of sodium hypochlorite and/or potassium hypochlorite at temperatures between 25°C and 110°C and then acidifying the solution using a strong inorganic acid.

2. The process as claimed in claim 1, wherein the reaction is carried out at temperatures between 28°C and 60°C.

3. A process for the preparation of an aqueous solution of the sodium and/or potassium salt of 4-(2′-chloroethyl)-benzoic acid from 4-(2′-chloroethyl)acetophenone, which process comprises reacting 4-(2′-chloroethyl)acetophenone with an aqueous alkaline solution of sodium hypochlorite and/or potassium hypochlorite at temperatures between 25°C and 110°C.

4. The process as claimed in claim 3, wherein the reaction is carried out at temperatures between 28°C and 60°C.

**Revendications**

1. Procédé de préparation de l'acide (chloro-2′éthyl)-4 benzoïque à partir de la (chloro-2′éthyl)-4 acétophénone, procédé caractérisé en ce qu'on fait réagir la (chloro-2′éthyl)-4 acétophénone avec une solution alcaline aqueuse d'hypochlorite de sodium et/ou de l'hypochlorite de potassium à des températures comprises entre 25°C et 110°C, puis l'on acidifie la solution à l'aide d'un acide minéral fort.

2. Procédé selon la revendication 1, caractérisé en ce qu'on conduit la réaction à des températures comprises entre 28°C et 60°C.

3. Procédé de préparation d'une solution aqueuse du sel de sodium et/ou de potassium de l'acide (chloro-2′éthyl)-4 benzoïque à partir de la (chloro-2′éthyl)-4 acétophénone, procédé caractérisé en ce qu'on fait réagir la (chloro-2′éthyl)-4 acétophénone avec une solution alcaline aqueuse d'hypochlorite de sodium et/ou d'hypochlorite de potassium à des températures comprises entre 25°C et 110°C.

4. Procédé selon la revendication 3, caractérisé en ce qu'on conduit la réaction à des températures comprises entre 28°C et 60°C.